(19) Europäisches Patentamt
European Patent Office
Office européen des brevets

(11) Veröffentlichungsnummer: **0 290 905 A1**

## (12) EUROPÄISCHE PATENTANMELDUNG

(21) Anmeldenummer: **88107012.2**

(22) Anmeldetag: **02.05.88**

(51) Int. Cl.4: **C07C 49/557 , C07C 45/29 , C07C 29/136 , C07C 33/05**

(30) Priorität: **13.05.87 DE 3715889**
**28.07.87 DE 3724877**

(43) Veröffentlichungstag der Anmeldung:
**17.11.88 Patentblatt 88/46**

(84) Benannte Vertragsstaaten:
**AT BE CH DE ES FR GB IT LI NL SE**

(71) Anmelder: **BAYER AG**
**Konzernverwaltung RP Patentabteilung**
**D-5090 Leverkusen 1 Bayerwerk(DE)**

(72) Erfinder: **Franckowiak, Gerhard, Dr.**
**Henselweg 10**
**D-5600 Wuppertal 1(DE)**
Erfinder: **Heine, Hans-Georg, Dr.**
**Am Heckerhof 14**
**D-4150 Krefeld(DE)**
Erfinder: **Samaan, Samir, Dr.**
**Paul-Ehrlich-Strasse 21**
**D-5600 Wuppertal 1(DE)**
Erfinder: **Wintel, Thomas, Dr.**
**Viktoriastrasse 44**
**D-5600 Wuppertal 1(DE)**

# (54) Substituierte Propargylketone mit geringem Isomerenanteil deren Herstellung aus Homopropargylalkoholen durch Oxidation und deren Verwendung zur Prostaglandinsynthese sowie die Herstellung von Homopropargylalkoholen aus Propargylketonen.

(57) Die Erfindung betrifft substituierte Propargylketone der Formel III

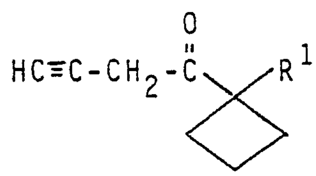

(III)

in der $R^1$ für geradkettiges oder verzweigtes Alkyl steht, mit geringem Isomerenanteil, deren Herstellung aus Homopropargylalkoholen durch Oxidation und deren Verwendung zur Prostaglandinsynthese sowie die Herstellung von Homopropargylalkoholen aus Propargylketonen.

EP 0 290 905 A1

# Substituierte Propargylketone mit geringem Isomerenanteil, deren Herstellung aus Homopropargylalkoholen durch Oxidation und deren Verwendung zur Prostaglandinsynthese sowie die Herstellung von Homopropargylalkoholen aus Propargylketonen

Die Erfindung betrifft substituierte Propargylketone, Verfahren zu ihrer Herstellung und ihre Verwendung als Synthesezwischenprodukte, insbesondere zur Synthese von Prostaglandinen.

In EP-PS 2590 und US-PS 4 132 738 sind physiologisch hochwirksame Prostaglandinderivate der Formel I genannt

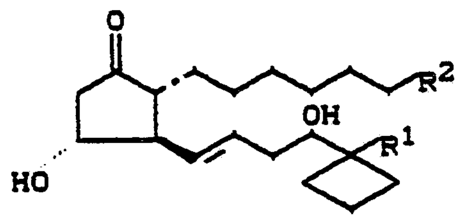

I

in welcher
$R^1$ für geradkettiges oder verzweigtes Alkyl und
$R^2$ für Hydroxymethyl oder für Alkoxycarbonyl steht.

Die dort beschriebenen Verfahren zur Herstellung der Prostaglandine benötigen die Homopropargylalkohole der Formel II,

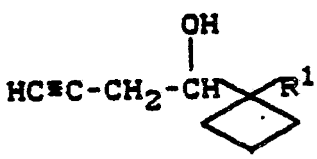

II

in welcher
$R^1$ die oben angegebene Bedeutung besitzt,
als Zwischenprodukte in racemischer wie auch in enantiomerenreiner Form.

Hierbei sind die R-Konfigumeren von besonderem Interesse.

Die bei der Racematspaltung anfallenden und nicht zur Synthese der Prostaglandinderivate der Formel I verwendeten Enantiomere der Formel II, insbesondere die S-Konfigumeren, stehen nach Racemisierung zur erneuten Racematspaltung zur Verfügung und sind damit Teil eines Recyclingverfahrens.

Unter verschiedenartigen Reaktionsbedingungen zur Racemisierung neigen Alkohole der allgemeinen Formel II, in denen die Hydroxylfunktion Teil eines Homopropargylsystems ist, zur Umlagerung in die entsprechenden Allene, die wiederum in Propargylalkohole übergehen können. Andererseits ist in Verbindungen der Formel II die Hydroxylfunktion auch Teil eines cyclischen Neopentylsystems, das zu Ringumlagerungsreaktionen neigt.

Die Erfindung betrifft ein Verfahren zur Herstellung von Propargylketonen der Formel III

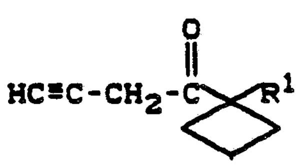

III

in welcher
$R^1$ die oben angegebene Bedeutung besitzt,
welches dadurch gekennzeichnet ist,
daß man Homopropargylalkohole der Formel II,
in welcher $R^1$ die oben angegebene Bedeutung besitzt, mit Oxidationsmitteln in Gegenwart inerter Lösemittel oxidiert.

Überraschenderweise können nach dem erfindungsgemäßen Verfahren die Propargylketone der Formel III mit nur geringem Isomerenanteil auf einfache und wirtschaftliche Weise hergestellt werden.

$R^1$ steht vorzugsweise für geradkettiges oder verzweigtes Alkyl mit bis zu 8 Kohlenstoffatomen, besonders bevor zugt mit bis zu 4 Kohlenstoffatomen. Ganz besonders bevorzugt steht $R^1$ für Methyl, Ethyl, n-Propyl oder n-Butyl.

Die als Ausgangsstoffe eingesetzten Homopropargylalkohole der allgemeinen Formel II sind bekannt und können nach bekannten Methoden hergestellt werden (US-PS 4 132 738).

Als Oxidationsmittel können Mangandioxid, Alkali-oder Erdalkalimanganate oder -permanganate sowie Verbindungen des 6-wertigen Chroms eingesetzt werden. Vorzugsweise werden Pyridiniumchlorchromat, Pyridiniumdichromat, Alkali-oder Erdalkalichromate oder -dichromate, insbesondere Natrium-oder Kaliumchromat, Natrium-oder Kaliumdichromat, sowie Chromsäure, insbesonders Chromtrioxid verwendet.

Als Lösemittel können Wasser und alle inerten organischen Lösemittel verwendet werden, die sich unter den Reaktionsbedingungen nicht verändern, Hierzu gehören bevorzugt Ether wie Diethylether, Diisobutylether, Diisopropylether, Dioxan oder Tetrahydrofuran, Ester wie Methyl-, Ethyl-, Propyl-oder Butylacetate, Eisessig, Acetonitril, halogenierte Kohlenwasserstoffe wie Methylenchlorid, Chloroform, Tetrachlorkohlenstoff, 1,1,1-Trichlorethan oder 1,1,2-Trichlortrifluorethan oder Kohlenwasserstoffe wie Benzol, Toluol, Xylol, Pentan, Hexan, Cyclohexan, Petrolether, Ligroin oder andere Alkanraktionen. Ebenso können Gemische oder mehrphasige Mischungen der genannten Lösemittel eingesetzt werden.

Die Reaktionstemperaturen können in einem größeren Bereich variiert werden. Im allgemeinen arbeitet man in einem Bereich von -80° C bis +100° C, bevorzugt von -60° C bis +60° C, insbesondere von -40° C bis +30° C.

Die Umsetzung kann bei Normaldruck, aber auch bei erhöhtem oder erniedrigtem Druck durchgeführt werden. Im allgemeinen arbeitet man bei Normaldruck. Die Reaktion kann unter einem Inertgas wie Stickstoff oder Argon durchgeführt werden.

Bei der Durchführung des erfindungsgemäßen Verfahrens ist das Verhältnis der an der Reaktion beteiligten Stoffe beliebig. Im allgemeinen arbeitet man mit äquivalenten Mengen der Reaktanden. Gegebenenfalls werden Säure-Katalysatoren wie Schwefelsäure, Phosphorsäure, Salzsäure, p-Toluolsulfonsäure oder stark saure Ionenaustauscher zugesetzt.

Verwendet man bei dem erfindungsgemäßen Verfahren als Edukt beispielsweise 1-(1-Hydroxybut-3-inyl)-1-n-butylcyclobutan in Petrolether und schwefelsaure wäßrige Chromsäurelösung, so läßt sich der Ablauf durch folgendes Formelschema beschreiben:

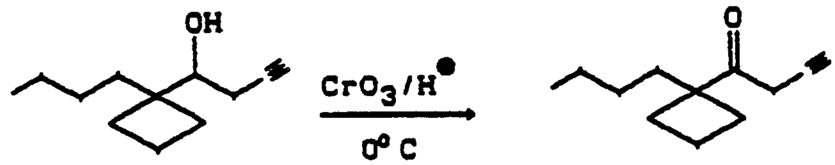


Die nach dem erfindungsgemäßen Verfahren gut zugänglichen Propargylketone sind wichtige Zwischenprodukte zur Herstellung von physiologisch hochwirksamen Prostaglandinderivaten. Hierzu werden die Ketone wieder zu den Homopropargylalkoholen der Formel II reduziert. Hierzu werden die literaturbekannten Methoden mit komplexen Hydriden angewendet.

Der Ablauf wird durch folgendes Formelschema beispielhaft beschrieben:

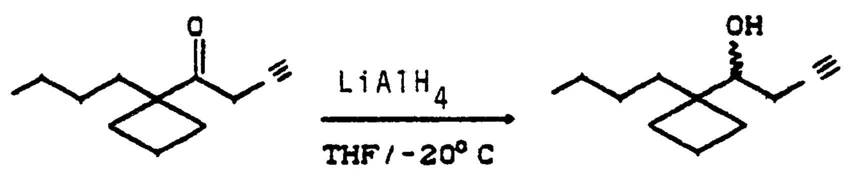


Als Reduktionsmittel können Alkali-oder Erdalkaliborhydride wie Natriumborhydrid, Natriumcyanoborhydrid, Tetramethylammoniumborhydrid, sowie Aluminiumhydride wie Lithiumaluminiumhydrid, oder Diisobutylaluminiumhydrid eingesetzt werden.

Als Lösemittel können alle inerten organischen Lösemittel verwendet werden, die sich unter den Reaktionsbedingungen nicht verändern. Hierzu gehören bevorzugt Ether wie Diethylether, Diisopropylether, Dioxan oder Tetrahydrofuran, halogenierte Kohlenwasserstoffe wie 1,1,2-Trichlortrifluorethan oder Kohlenwasserstoffe wie Benzol, Toluol, Xylol, Pentan, Hexan, Cylcohexan, Petrolether, Isohexan, Ligroin oder andere Alkanfraktionen. Ebenso können Gemische oder mehrphasige Mischungen der genannten Lösemittel eingesetzt werden.

Die Reaktionstemperaturen können in einem größeren Bereich variiert werden. Im allgemeinen arbeitet man in einem Bereich von -80° C bis +100° C, bevorzugt von -60° C bis +60° C, insbesondere von -40° C bis +30° C.

Die Umsetzung kann bei Normaldruck, aber auch bei erhöhtem oder erniedrigtem Druck durchgeführt werden. Im allgemeinen arbeitet man bei Normaldruck oder leichtem Überdruck. Die Reaktion kann unter einem Inertgas wie Stickstoff oder Argon durchgeführt werden.

Bei der Durchführung des erfindungsgemäßen Verfahrens ist das Verhältnis der an der Reaktion beteiligten Stoffe beliebig. Im allgemeinen arbeitet man mit äquivalenten Mengen der Reaktanden.

Beispiel 1

Zu 1150 g S-(1-Hydroxybut-3-inyl)-1-propyl-cyclobutan in 6,8 1 Petrolether läßt man unter Kühlung bei 0-5° C langsam eine kalte Lösung von 1120 g Chromsäure und 1030 g konz. Schwefelsäure in 8,5 1 Wasser zulaufen und 10 Stunden bei 0° C nachreagieren.

Nach Phasentrennung extrahiert man die wäßrige Phase mehrfach mit Petrolether, wäscht die organische Phase mit Wasser und trocknet sie mit Natriumsulfat. Nach Abdampfen des Lösungsmittels im Vakuum kann das rohe 1-(1-Oxo-but-3-inyl)-1-propyl-cyclobutan (Ausbeute: 1093 g) direkt reduziert werden (Beispiel 2).

Nach Destillation erhält man das reine Keton XXVI als farblose Flüssigkeit.
Ausbeute: 997,4 g (87,8 % d.Th.)
Sdp. $_{0,75\ mbar}$ 55-59° C
analog werden erhalten:
1-Methyl-1-(1-oxo-but-3-inyl)-cyclobutan
1-Ethyl-1-(1-oxo-but-3-inyl)-cyclobutan
1-Isopropyl-1-(1-oxo-but-3-inyl)-cyclobutan
1-n-Butyl-1-(1-oxo-but-3-inyl)-cyclobutan

Beispiel 2

In einem 50 1-Autoklaven werden 225 g Lithiumaluminiumhydrid in 18 1 Ether vorgelegt. Unter Kühlung auf -20° C werden 1,9 kg rohes Keton aus Beispiel 1 in 2,5 1 Ether eindosiert. Man läßt 1 Stunde bei -20° C und über Nacht bei Raumtemperatur ausreagieren und hydrolysiert durch Zugabe von überschüssiger gesättigter Ammoniumchloridlösung. die wäßrige Phase wird mehrfach mit Ether extrahiert. Die vereinigten organischen Phasen dampft man nach Trocknen im Vakuum ein und erhält das racemische 1-(1-Hydroxybut-3-inyl)-1-propyl-cyclobutan nach Destillation als farblose Flüssigkeit. Ausbeute 1790 g (94 % d.Th.)
sdp. $_{15\ mbar}$ 98-104° C

## Ansprüche

1. Verfahren zur Herstelung von Propargylketonen der Formel III

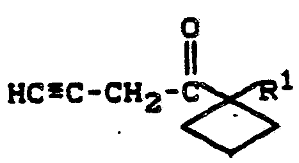

(III)

in der
$R^1$ für geradkettiges oder verzweigtes Alkyl steht,
dadurch gekennzeichnet, daß man Homopropargylalkohole der Formel II

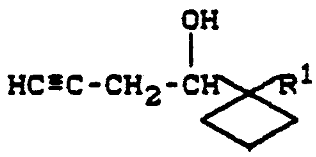

(II)

mit Oxationsmitteln oxidiert.

2. Proparglylketone der Formel III

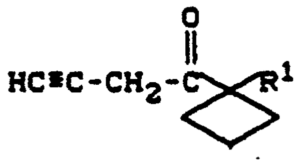

(III)

in der
$R^1$ für geradkettiges oder verzweigtes Alkyl steht.

3. Verwendung von Verbindungen der Formel III nach Anspruch 2 zur Herstellung von Prostaglandinen.

4. Verfahren zur Herstellung von Homopropargylalkoholen der Formel II

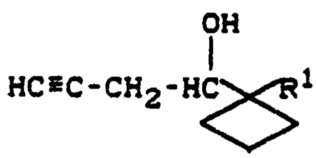

(II)

in der
$R^1$ für geradkettiges oder verzweigtes Alkyl steht,
dadurch gekennzeichnet, daß man Propargylketone der Formel III

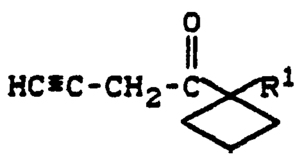

(III)

in der
$R^1$ die oben angegebene Bedeutung hat,
in einem inerten organischen Lösungsmittel bei Temperaturen von -80° C bis +100° C reduziert.

Europäisches Patentamt

# EUROPÄISCHER RECHERCHENBERICHT

Nummer der Anmeldung

EP 88 10 7012

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| Y | US-A-4 312 994 (P.W. COLLINS)<br>* Spalte 12, Beispiel 35 *<br>--- | 1-3 | C 07 C 49/557<br>C 07 C 45/29<br>C 07 C 29/36<br>C 07 C 33/05 |
| D,Y | US-A-4 132 738 (H.C. KLUENDER et al.)<br>* Spalte 43, Zeile 54-65 * | 1-3 | |
| D,A | * Spalte 31, Zeile 2; Spalte 27, Zeile7; Spalte 29, Zeile 34 *<br>--- | 1 | |
| A | M. HUDLICKY: "Reductions in organic chemistry", Seiten 119-122, Ellis Horwood Ltd., Chichester, 1984<br>* Seite 122 *<br>--- | 4 | |
| A | US-A-2 779 799 (K.E. HAMLIN)<br>* Anspruch *<br>----- | 4 | |
| | | | **RECHERCHIERTE SACHGEBIETE (Int. Cl.4)** |
| | | | C 07 C 49/00<br>C 07 C 33/00<br>C 07 C 29/00 |

Der vorliegende Recherchenbericht wurde für alle Patentansprüche erstellt

| Recherchenort | Abschlußdatum der Recherche | Prüfer |
|---|---|---|
| BERLIN | 22-07-1988 | PROBERT C.L. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)